(19) Europäisches Patentamt
European Patent Office
Office européen des brevets

(11) **EP 4 300 062 B1**

(12) **EUROPEAN PATENT SPECIFICATION**

(45) Date of publication and mention
of the grant of the patent:
**18.12.2024 Bulletin 2024/51**

(21) Application number: **23159075.3**

(22) Date of filing: **28.02.2023**

(51) International Patent Classification (IPC):
*G01K 13/20* (2021.01)     *G01K 7/42* (2006.01)
*A61B 5/01* (2006.01)     *A61B 5/00* (2006.01)

(52) Cooperative Patent Classification (CPC):
**G01K 13/20; A61B 5/0008; A61B 5/01;**
**G01K 7/427**

(54) **ELECTRONIC DEVICE AND METHOD OF ESTIMATING BODY TEMPERATURE USING THE SAME**

ELEKTRONISCHE VORRICHTUNG UND VERFAHREN ZUR SCHÄTZUNG DER KÖRPERTEMPERATUR DAMIT

DISPOSITIF ÉLECTRONIQUE ET PROCÉDÉ D'ESTIMATION DE LA TEMPÉRATURE CORPORELLE L'UTILISANT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.06.2022 KR 20220078932**
**08.02.2023 KR 20230016885**

(43) Date of publication of application:
**03.01.2024 Bulletin 2024/01**

(73) Proprietor: **Samsung Electronics Co., Ltd.**
**Suwon-si, Gyeonggi-do 16677 (KR)**

(72) Inventors:
• **Kim, Sung Ho**
**Suwon-si, Gyeonggi-do (KR)**
• **Lee, So Young**
**Suwon-si, Gyeonggi-do (KR)**
• **Kwon, Bok Soon**
**Suwon-si, Gyeonggi-do (KR)**
• **Kim, Sang Kyu**
**Suwon-si, Gyeonggi-do (KR)**
• **Lee, Ho Taik**
**Suwon-si, Gyeonggi-do (KR)**
• **Rhee, Hong Soon**
**Suwon-si, Gyeonggi-do (KR)**

(74) Representative: **Grünecker Patent- und Rechtsanwälte**
**PartG mbB**
**Leopoldstraße 4**
**80802 München (DE)**

(56) References cited:
**JP-A- S61 120 026     US-A1- 2022 042 856**

**Description**

BACKGROUND

**1. Field**

Apparatuses and methods consistent with example embodiments relate to using temperature sensors for estimating body temperature in a wearable device and/or the sensor device.

**2. Description of the Related Art**

Generally, body temperature is one of four vital signs and has very important clinical significance. A body temperature sensor may be applied to various applications, such as checking infections in patients, thermal side effects of medications, or time of ovulation in women, and the like. However, due to the size of a body temperature sensor, it may be difficult to measure the body temperature by using a portable device such as a wearable device. A heat flux sensor used in a portable device for body temperature estimation measures the heat flux by using two or more temperature sensors and an insulator for compensating for body heat. In this case, in order to accurately measure the heat flux, it is required to reduce noise generated during an analog-to-digital (ADC) conversion process for reading sensor data. To this end, it is desirable to obtain a large difference between temperatures measured by the temperature sensors. Generally, in order to obtain a large temperature difference, the thickness of the insulator may be increased, but the increased thickness of the insulator may lead to an increase in thickness of the heat flux sensor, thereby causing a problem in that it is difficult to manufacture the portable device in a compact size.

JP 561120026 A refers to a deep thermometer using a non-heat flow compensation method. A heat insulating material has a relatively low thermal conductivity in contact with a surface of a body. A temperature sensor is arranged at a center position of the heat insulating material contact surface with a living body, and an air contact facing the contact surface of the heat insulating material.

US 2022/042856 A1 refers to a temperature sensor unit and body core thermometer. The temperature sensor unit is used to measure a deep part body temperature Ti as a body core temperature. The temperature sensor unit comprises at a measurement face side facing a body surface first-fourth temperature sensors for measuring the body surface. Among the first and the second temperature sensors, the first thermal resistor is disposed only at the measurement face side of the first temperature sensor. Furthermore, the first temperature sensor and the second temperature sensors are disposed proximally such that a temperature Ti at the measurement face side of the first thermal resistor becomes approximately equal to a temperature T2 measured by the second temperature sensor.

SUMMARY

The invention is claimed by the independent claims. Preferred embodiments are specified in the dependent claims.

BRIEF DESCRIPTION OF THE DRAWINGS

Aspects of the invention will be more apparent by describing certain example embodiments, with reference to the accompanying drawings, in which:

FIG. 1 is a block diagram illustrating an electronic device according to an embodiment of the present disclosure;
FIG. 2 is a block diagram illustrating a heat flux sensor according to an embodiment of the present disclosure;
FIG. 3 is a diagram illustrating a structure of a heat flux sensor according to an embodiment of the present disclosure;
FIG. 4 is a diagram illustrating a circuit structure of a heat flux sensor according to an embodiment of the present disclosure;
FIG. 5 is a diagram explaining an example of estimating a user's body temperature;
FIG. 6 is a block diagram illustrating an electronic device according to another embodiment of the present disclosure;
FIG. 7 is a flowchart illustrating a method of estimating body temperature according to an embodiment of the present disclosure; and
FIGS. 8 to 13 are diagrams illustrating examples of structures of an electronic device.

DETAILED DESCRIPTION

Example embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

It will be understood that, although the terms first, second, etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'unit' or 'module', etc., should be understood as a unit that performs at least one function or operation and that may be embodied as hardware, software, or a combination thereof.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

It will be understood that, although in the following reference is provided to measure a heat flux and/or to use a heat flux sensor, all of the embodiments of the present invention as described in this disclosure can be realized without measuring a heat flux and/or without using a heat flux sensor. As an alternative, temperature sensors can be used that are configured to measure voltages when the sensors are in contact with a user. The achieved voltages are further processed e.g. by amplifying a voltage difference and by transferring amplified voltages e.g. the voltage difference from an analog format to a digital format. One or more processors are further used to estimate a body temperature of the user based on digital signals representing the amplified voltage difference.

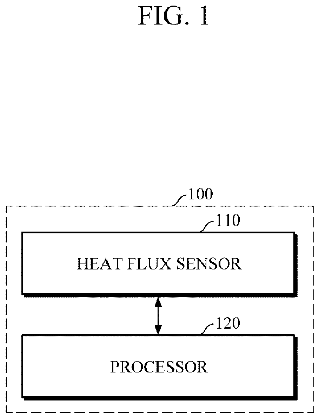

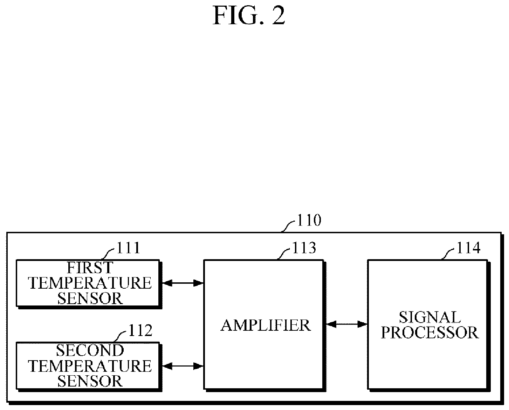

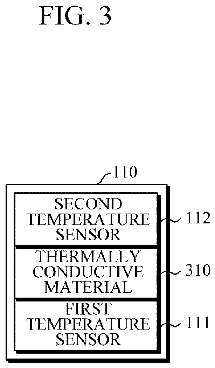

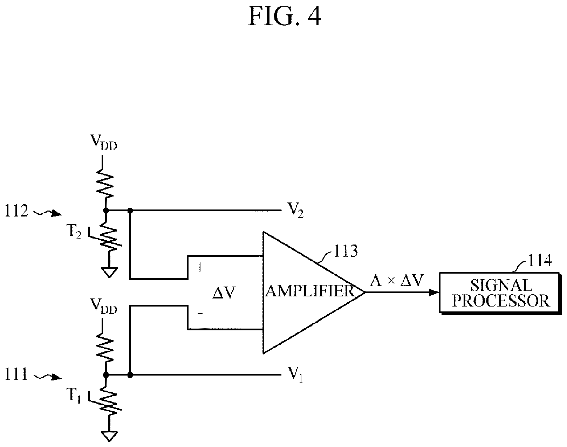

FIG. 1 is a block diagram illustrating an electronic device according to an embodiment of the present disclosure. FIG. 2 is a block diagram illustrating a heat flux sensor according to an embodiment of the present disclosure. FIG. 3 is a diagram illustrating a structure of a heat flux sensor according to an embodiment of the present disclosure. FIG. 4 is a diagram illustrating a circuit structure of a heat flux sensor according to an embodiment of the present disclosure.

Referring to FIG. 1, an electronic device 100 includes a heat flux sensor 110 and a processor 120.

The heat flux sensor 110 may include a plurality of sensors and circuit elements for obtaining data for estimating a user's body temperature, and the processor 120 may estimate body temperature by using the obtained data. The processor 120 may be electrically connected to the heat flux sensor 110 and may control the heat flux sensor 110 in response to a request for estimating body temperature.

Referring to FIGS. 2 and 3, the heat flux sensor 110 may include a first temperature sensor 111, a second temperature sensor 112, an amplifier 113, and a signal processor 114. In addition, the first temperature sensor 111 and the second temperature sensor 112 may be formed in a stacked structure with a thermally conductive material 310 disposed therebetween. In this case, the thermally conductive material 310 may be a thermally insulating material. Although FIG. 2 illustrates that the first temperature sensor 111, the second temperature sensor 112, and the signal processor 114 are included in the heat flux sensor 110, embodiments are not limited thereto. As an alternative, the first temperature sensor 111 and the second temperature sensor 112 may be used to directly measure a first and a second voltage, respectively, when in contact with the user, and the signal processor 114 may be incorporated into the processor 120.

The first temperature sensor 111 may measure a first temperature in the electronic device 100. The second temperature sensor 112 may be spaced apart from the first temperature sensor 111 and may measure a second temperature in the electronic device 100. For example, the first temperature sensor 111 may be disposed at a lower end of the electronic device 100 to measure a surface temperature of a contact surface between the electronic device 100 and an object. When the heat flux sensor 110 is in contact with a user, the first temperature sensor 111 may measure a skin temperature of the user. In this case, the object may be a body part (e.g., forehead, chest, earlobe, upper arm, wrist, etc.) where body temperature may be well reflected, but is not limited thereto.

The thermally conductive material 310 may be an insulator having a size of 0.1 mm to 5 mm, and may be a material (e.g., polyurethane foam or air) having a thermal conductivity of 0.1 W/mK or less. However, the size and thermal conductivity of the insulator is not limited thereto. Further, an air-filled structure may also be provided in which air having a very low thermal conductivity is filled between the first temperature sensor 111 and the second temperature sensor 112, without using a separate material. The first temperature sensor 111 and the second temperature sensor 112 may be thermistors. Among temperature sensors for measuring temperature, the thermistor is a contact-type temperature sensor and may come into contact with, for example, the wrist of an object to measure a surface temperature of the wrist. In addition, the first temperature sensor 111 and the second temperature sensor 112 may be arranged as a thermistor pair with the thermally conductive material 310 disposed therebetween.

The amplifier 113 may be a circuit element having one or more input terminals and amplifying a voltage difference of an input signal input through the input terminals, and may be for example, a differential amplifier. However, the type of the amplifier 113 is not limited thereto. Referring to FIG. 4, for example, the amplifier 113 may amplify a voltage difference $\Delta V$ between a first voltage Vi, measured by the first temperature sensor 111, and a second voltage $V_2$ measured by the second temperature sensor 112, to generate the amplified voltage difference A*$\Delta V$. In this case, the first temperature sensor 111 and the second temperature sensor 112 may have the same or substantially the same circuit structure so that the voltage difference $\Delta V$ may be easily obtained. For example, the first temperature sensor 111 and the second temperature sensor 112 may be arranged in a Wheatstone bridge configuration around the amplifier 113. However, the arrangement of the first temperature sensor 111 and the second temperature sensor 112 is not limited thereto.

The signal processor 114 may convert the amplified voltage difference into a temperature difference, and may calculate heat flux based on the converted temperature difference to output the heat flux. For example, the signal processor 114 may convert the voltage difference into the temperature difference by preprocessing the amplified voltage difference and inputting the voltage difference to a predetermined conversion model.

First, the signal processor 114 may perform preprocessing on the amplified voltage difference A*$\Delta V$. For example, the signal processor 114 may divide the amplified voltage difference A*$\Delta V$ by a predetermined value, thereby alleviating the problem of decreasing resolution during an analog-to-digital (ADC) conversion process. The signal processor 114 may include any one or any combination of a digital circuit, an analog circuit, and an ADC converter.

Then, by inputting the amplified and preprocessed voltage difference to a predetermined conversion model, the signal processor 114 may convert the voltage difference into the temperature difference. In particular, the signal processor 114 may generate the conversion model based on the first temperature and the first voltage or the second temperature and the second voltage, and an external supply voltage.

Referring to FIG. 4, for example, assuming that the first temperature sensor 111 and the second temperature sensor 112 are thermistors having the same circuit, the first temperature $T_1$ measured by the first temperature sensor 111 may be represented by the following Equation 1. In this case, for example, a resistance value of a resistor directly connected to the external supply voltage may be a resistance value at the time when the first temperature sensor 111 and the second temperature sensor 112 are at 25 °C (298K). However, the resistance value is not limited thereto.

[Equation 1]

$$T_1 = \left[ \frac{\ln\left(\frac{V_1}{V_{DD} - V_1}\right)}{B} + \frac{1}{298} \right]^{-1}$$

Herein, $V_{DD}$ denotes the external supply voltage supplied to the electronic device, and B denotes a characteristic value of a thermistor.

In this case, the following Equation 2 may be obtained by differentiating the first temperature $T_1$ of Equation 1 with respect to Vi.

[Equation 2]

$$\frac{dT_1}{dV_1} = -\left[ \frac{\ln\left(\frac{V_1}{V_{DD} - V_1}\right)}{B} + \frac{1}{298} \right]^{-2} \left[ \frac{1}{B}\left(\frac{V_{DD} - V_1}{V_1}\right)\left(\frac{V_{DD}}{(V_{DD} - V_1)^2}\right) \right]$$

$$= -T_1^2 \left[ \frac{1}{B}\left(\frac{V_{DD}}{V_1(V_{DD} - V_1)}\right) \right]$$

If a temperature difference between the first temperature $T_1$ and the second temperature $T_2$ is small, the temperature difference may be approximated as shown in Equation 3, and a final conversion model may be generated according to Equation 3.

[Equation 3]

$$\Delta T = -T_1^2 \left[ \frac{1}{B} \left( \frac{V_{DD}}{V_1(V_{DD} - V_1)} \right) \right] \Delta V$$

Herein, $\Delta V$ denotes the voltage difference between the voltage Vi measured by the first temperature sensor 111 and the voltage $V_2$ measured by the second temperature sensor 112, and $\Delta T$ denotes the temperature difference between the first temperature $T_1$ and the second temperature $T_2$.

In this case, by substituting the amplified and preprocessed voltage difference $\Delta V_p$ into the voltage difference $\Delta V$ of Equation 3, the voltage difference may be converted into a relatively large temperature difference $\Delta T_p$.

In order to accurately measure body temperature, it may be desirable to obtain a large difference between temperatures measured by temperature sensors, to increase heat flux generated by the temperature difference. However, in order to obtain a large temperature difference, a problem occurs in that it is required to increase the volume of a device itself. However, by obtaining a large temperature difference using the amplified voltage difference according to this embodiment of the present disclosure, not only the device may be manufactured in a compact size, but also the accuracy of estimating body temperature may be improved.

In this embodiment, the conversion model is generated based on the first temperature $T_1$ measured by the first temperature sensor 111, but the same method may also be applied to the case where the conversion model is generated based on the second temperature $T_2$ measured by the second temperature sensor 112.

Then, the signal processor 114 may calculate the heat flux based on the converted temperature difference and may output the heat flux. For example, the signal processor 114 may obtain the converted temperature difference $\Delta T_p$ by substituting the amplified and preprocessed voltage difference $\Delta V_p$ into the above Equation 3, and may calculate the heat flux according to the following Equation 4 by applying a thermal coefficient of resistivity of the thermally conductive material to the converted temperature difference $\Delta T_p$.

[Equation 4]

$$HF = \beta_{ins}\Delta T_p$$

Herein, HF denotes the heat flux, and $\beta$ins denotes a predetermined thermal coefficient of resistivity of the thermally conductive material 310.

Subsequently, the processor 120 may estimate a user's body temperature based on the heat flux output by the signal processor 114.

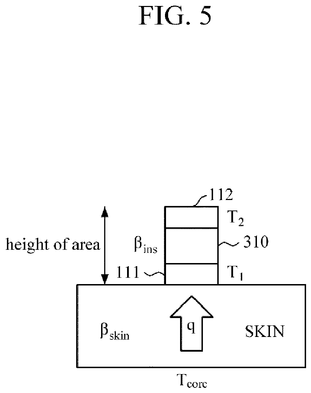

FIG. 5 is a diagram explaining an example of estimating a user's body temperature.

Referring to FIG. 5, a difference between body temperature $T_{core}$ of a user and a surface temperature $T_1$ of an object may be represented as heat flux q in the following Equation 5.

[Equation 5]

$$q = \beta_{skin}(T_{core} - T_1)$$

Herein, $\beta_{skin}$ denotes a predetermined skin heat transfer coefficient.

In this case, assuming that heat transfer from the core occurs in a series circuit, the heat flux q is equal to the heat flux HF output by the signal processor 114 of the heat flux sensor 110, which may be represented by the following Equation 6.

[Equation 6]

$$\beta_{skin}(T_{core} - T_1) = \beta_{ins}\Delta T_p$$

Equation 6 may be rearranged as the following Equation 7.

[Equation 7]

$$T_{core} = T_1 + \frac{\beta_{ins}\Delta T_p}{\beta_{skin}} = T_1 + \frac{HF}{\beta_{skin}}$$

According to the above Equation 7, the processor 120 may estimate the body temperature based on the heat flux HF and the first temperature $T_1$ which is the surface temperature of the object.

Referring to FIG. 5, the first temperature sensor 111 and the second temperature sensor 112 are spaced apart from each other with a space therebetween. The space may be fully filled with the thermally conductive material 310, or partially filled with the thermally conductive material 310. When the space between the first temperature sensor 111 and the second temperature sensor 112 is partially filled with the thermally conductive material 310, and there may be air between the thermally conductive material 310 and either one or both of the first temperature sensor 111 and the second temperature sensor 112. In such a case, the air and the thermally conductive material 310 together may act as an insulator.

The first temperature sensor 111, the second temperature sensor 112, and the thermally conductive material 310 may be provided in an area between a contact surface of a main body and a display panel of an electronic device 100. When the electronic device 100 is implemented as a smart watch, there may be restrictions on the height of each of the temperature sensors 111 and 112 and a distance between the temperature sensors 111 and 112 since the area of the smart watch that can accommodate the temperature sensors 111 and 112 is small. The distance between the temperature sensors 111 and 112 may correspond to the thickness of the thermally conductive material 310 when the space between the temperature sensors 111 and 112 is fully filled with the thermally conductive material 310.

For example, the height of the area of the smart watch that can accommodate the temperature sensors 111 and 112 may be in a range from 1 mm to 1.5 mm. Given the limited height of the area in the smart watch, the height of the thermally conductive material 310 may decrease as the height of the temperature sensors 111 and 112 increases, while a certain distance between the two temperature sensors 111 and 112 is required to obtain a minimum temperature difference (e.g., 0.3 °C) between the two temperature sensors 111 and 112 and thereby to estimate a body temperature based on the temperature difference. Since the temperature sensors 111 and 112 may have some error rate (e.g., ± 0.1 °C), it may be difficult to reliably measure the temperature difference between the two temperature sensors 111 and 112 when a target temperature difference between the two temperature sensors 111 and 112 is set to be less than 0.3 °C. Based on such understanding, a minimum target temperature difference between the two temperature sensors 111 and 112 may be set to 0.3 °C, and a heat transfer simulation has been conducted by changing the height of the temperature sensors 111 and 112 and the height of the thermally conductive material 310 (or the distance between the temperature sensors 111 and 112 when the space between the temperature sensors 111 and 112 is not fully filed with the thermally conductive material 310) for each of a plurality of area heights H, as shown below in Table 1.

Table 1

| Area height H | Height of Temperature Sensor | Height of Thermally Conductive Material (or Distance Between Temperature Sensors) | Temperature Difference |
|---|---|---|---|
| 1mm | 0.1 | 0.8 | 0.648 |

| | 0.2 | 0.6 | 0.486 |
|---|---|---|---|
| | 0.3 | 0.4 | 0.324 |
| | 0.4 | 0.2 | 0.162 |
| 1.1mm | 0.1 | 0.9 | 0.730 |
| | 0.2 | 0.7 | 0.567 |
| | 0.3 | 0.5 | 0.405 |
| | 0.4 | 0.3 | 0.243 |
| 1.2mm | 0.1 | 1 | 0.811 |
| | 0.2 | 0.8 | 0.648 |
| | 0.3 | 0.6 | 0.486 |
| | 0.4 | 0.4 | 0.324 |
| | 0.5 | 0.2 | 0.162 |
| 1.3mm | 0.1 | 1.1 | 0.892 |
| | 0.2 | 0.9 | 0.730 |
| | 0.3 | 0.7 | 0.567 |
| | 0.4 | 0.5 | 0.405 |
| | 0.5 | 0.3 | 0.243 |
| 1.4mm | 0.1 | 1.2 | 0.973 |
| | 0.2 | 1 | 0.811 |
| | 0.3 | 0.8 | 0.648 |
| | 0.4 | 0.6 | 0.486 |
| | 0.5 | 0.4 | 0.324 |
| | 0.6 | 0.2 | 0.162 |
| 1.5mm | 0.1 | 1.3 | 1.054 |
| | 0.2 | 1.1 | 0.892 |
| | 0.3 | 0.9 | 0.730 |
| | 0.4 | 0.7 | 0.567 |
| | 0.5 | 0.5 | 0.405 |
| | 0.6 | 0.3 | 0.243 |

Referring to Table 1 above, when a target temperature difference between the two temperature sensors 111 and 112 is greater than or equal to 0.3°C, the height of each of the temperature sensors 111 and 112 may be set to have a minimum height of 0.3 mm (i.e., 0.3 mm or greater, and preferably from 0.3 mm to 0.5 mm), and the height of the thermally conductive material 130 (or the distance between the temperature sensors 111 and 112) may be set to a minimum height of 0.4 mm or (i.e., 0.4 mm or greater, and preferably from 0.4 mm to 1.3 mm). In a case where the thermally conductive material 130 does not exist between the two temperature sensors 111 and 112 , and/or there is a space (e.g., air) in addition to the thermally conductive material 130 between the two temperature sensors 111 and 112, the height of the thermally conductive material 130 may refer to a distance between the two temperature sensors 111 and 112.

The first temperature sensor 111 may be disposed as close as possible to the contact surface, and the second temperature sensor 112 may be disposed as close as possible to the display panel to provide a relatively accurate temperature estimation.

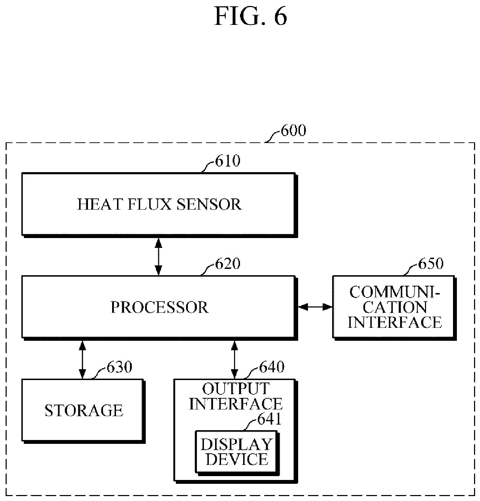

FIG. 6 is a block diagram illustrating an electronic device according to another embodiment of the present disclosure.

Referring to FIG. 6, an electronic device 600 may include a heat flux sensor 610, a processor 620, a storage 630, an output interface 640, and a communication interface 650. In this case, the output interface 640 may include a display device 641. The heat flux sensor 610 and the processor 620 are the same as the heat flux sensor 610 and the processor 620 in the embodiment of FIG. 1, such that a detailed description thereof will be omitted.

The storage 630 may store information related to estimating body temperature. For example, the storage 630 may store the first temperature, second temperature, amplified voltage difference, converted temperature difference, estimated heat flux, skin heat transfer coefficient, heat transfer coefficient of the thermally conductive material, and processing results of the processor 620, e.g., a user's body temperature and the like.

The storage 630 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The output interface 640 may provide the processing results of the processor 620 to a user. For example, the output interface 640 may display an estimated body temperature value of the processor 620 on the display device 641. In this case, if the estimated body temperature value falls outside a (predetermined) normal range, the output interface 640 may provide the user with warning information by changing color, line thickness, etc., or displaying an abnormal value along with the normal range, so that the user may easily recognize the abnormal value. In addition, along with or without the visual output displayed on the display device 641, the output interface 640 may provide the user with the first temperature, second temperature, body temperature, or body temperature guidance information in a non-visual manner by voice, vibrations, tactile sensation, and the like using an audio output module such as a speaker, or a haptic module and the like.

The display device 641 may include a display, a hologram device, or a projector and control circuitry to control the devices. The display device 641 may include touch circuitry adapted to detect a touch, and/or sensor circuitry (e.g., pressure sensor, etc.) adapted to measure the intensity of force incurred by the touch.

The audio module may convert a sound into an electrical signal or vice versa. The audio module may output the sound via a speaker and/or a headphone of another electronic device directly or wirelessly connected to the apparatus for estimating body temperature.

The haptic module may convert an electrical signal into a mechanical stimulus (e.g., vibration, motion, etc.) or electrical stimulus which may be recognized by a user by tactile sensation or kinesthetic sensation. The haptic module may include, for example, a motor, a piezoelectric element, and/or an electric stimulator.

The communication interface 650 may communicate with an external device to transmit and receive various data related to estimating body temperature. The external device may include an information processing device such as a smartphone, a tablet PC, a desktop computer, a laptop computer, and the like. For example, the communication interface 650 may transmit a body temperature estimation result to the external device, such as a user's smartphone and the like, so that the user may manage and monitor the estimation result by using a device having a relatively high performance.

The communication interface 650 may communicate with the external device by using various wired or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, third-generation (3G), fourth-generation (4G), fifth-generation (5G), and sixth-generation (6G) communications, and the like. However, this is merely exemplary and is not intended to be limiting.

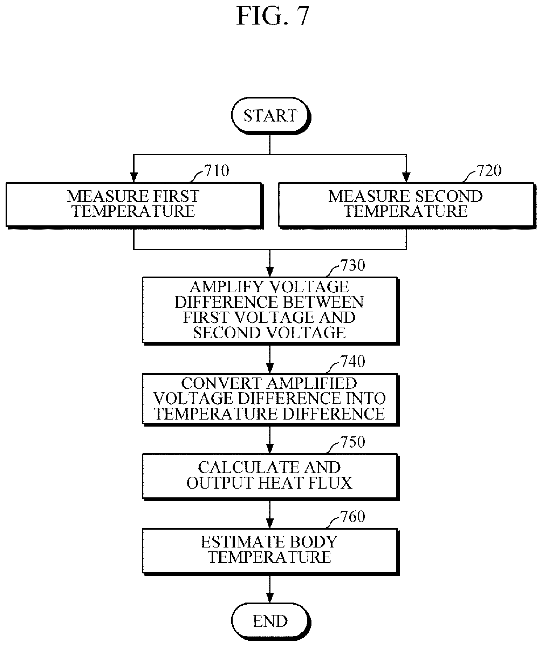

FIG. 7 is a flowchart illustrating a method of estimating body temperature according to an embodiment of the present invention.

The method of FIG. 7 is a method of estimating body temperature performed by the electronic devices 100 and 600 according to the embodiments of FIGS. 1 and 6, which are described in detail above, and thus will be briefly described below in order to avoid redundancy.

Referring to FIG. 7, the electronic device first measures a first temperature by using the first temperature sensor in operation 710, and measures a second temperature by using the second temperature sensor spaced apart from the first temperature sensor in operation 720. In this case, the first temperature may be the surface temperature of an object.

Then, the electronic device amplifies a voltage difference between a first voltage, measured by the first temperature sensor, and a second voltage measured by the second temperature sensor in operation 730. In this case, the first temperature sensor and the second temperature sensor may be arranged in a Wheatstone bridge configuration.

Subsequently, the electronic device converts the voltage difference, amplified by the signal processor, into a temperature difference in operation 740. In this case, the signal processor may convert the voltage difference into the temperature difference by preprocessing the amplified voltage difference and inputting the voltage difference to a predetermined conversion model. In this case, the signal processor may generate the conversion model based on at least either the first temperature and the first voltage or the second temperature and the second voltage, and an external supply voltage.

Next, the signal processor may calculate heat flux based on the converted temperature difference and may output the calculated heat flux in operation 750. In this case, the signal processor may calculate the heat flux by applying a thermal coefficient of resistivity of the thermally conductive material to the converted temperature difference.

Then, the processor estimates body temperature based on the output heat flux and the surface temperature of the object in operation 760. In this case, the processor may output the first temperature, second temperature, body temperature, or body temperature guidance information, and the like through the display of the output interface, to provide the information to a user.

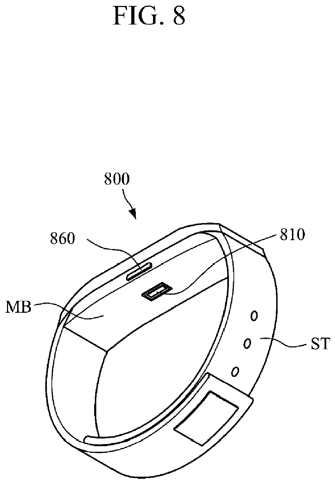

FIGS. 8 to 13 are diagrams illustrating examples of structures of an electronic device.

Referring to FIG. 8, the electronic device is implemented as a smart watch-type wearable device 800 which includes a main body MB and a wrist strap ST.

The main body MB may be formed in various shapes. A battery may be embedded in the main body MB and/or the strap ST to supply power to various components of the wearable device. The strap ST may be connected to both ends of the main body to allow the main body to be worn on a user's wrist, and may be flexible so as to be wrapped around the user's wrist. The strap ST may be composed of a first strap and a second strap which are separated from each other. One end of each of the first strap and the second strap is connected to both sides of the main body MB, and the first strap and the second strap may be connected to each other via a fastening means formed at the other end of the respective straps. In this case, the fastening means may be formed as magnetic connection, Velcro connection, pin connection, and the like, but is not limited thereto. Further, the strap ST is not limited thereto, and may be integrally formed as a non-detachable band.

The main body MB may include a heat flux sensor 810, a processor, an output interface, a storage, and a communication interface. However, depending on the size and shape of a form factor and the like, some of the output interface, the storage, and the communication interface may be omitted.

The heat flux sensor 810 may include: a first temperature sensor disposed in the main body and configured to measure a first temperature; a second temperature sensor spaced apart from the first temperature sensor and configured to measure a second temperature; an amplifier configured to amplify a voltage difference between a voltage measured by the first temperature sensor and a voltage measured by the second temperature sensor; and a signal processor configured to convert the amplified voltage difference into a temperature difference, and to calculate heat flux based on the converted temperature difference to output the calculated heat flux. In this case, the signal processor may convert the voltage difference into the temperature difference by preprocessing the amplified voltage difference and inputting the voltage difference to a predetermined conversion model.

The processor mounted in the main body MB may be electrically connected to various components including the heat flux sensor 810. The processor may estimate a user's body temperature based on the heat flux output by the heat flux sensor 810. For example, the processor may estimate the body temperature based on the output heat flux and the first temperature.

A manipulator 860 may be formed on a side surface of the main body MB, as illustrated herein. The manipulator 860 may receive a user's command and may transmit the received command to the processor. In addition, the manipulator 860 may have a power button to turn on/off the wearable device 800.

A display may be provided on a front surface of the main body MB, and may display various application screens including body temperature information, time information, received message information, and the like. For example, the processor may display an estimated body temperature value on the display. In this case, if an estimated body temperature value falls outside a normal range, the processor may provide the user with warning information by changing color, line thickness, etc., or displaying an abnormal value along with the normal range, so that the user may easily recognize the abnormal value. In addition, in response to the user's request, the processor may display and provide not only a current estimated body temperature value, but also continuous estimated body temperature values over time on the display for the user. In addition, the processor may display a body temperature variation, for example, a body temperature change during a day, in a graph on the display, and may also display information on sleep quality according to the body temperature change on the display. The information which may be displayed on the display may include not only the body temperature information, but also the first temperature, second temperature, body temperature guidance information, etc., but is not limited thereto.

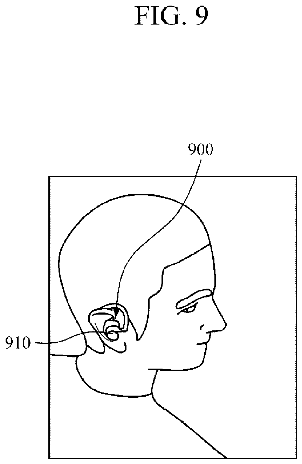

Referring to FIG. 9, the electronic device may be implemented as an ear-wearable device 900.

The ear-wearable device 900 may include a main body and an ear strap. A user may wear the ear-wearable device 900 by hanging the ear strap on the user's auricle. The ear strap may be omitted depending on the shape of the ear-wearable device 900. The main body may be inserted into the external auditory meatus. A heat flux sensor 910 may be mounted in the main body. The ear-wearable device 900 may provide the user with a body temperature estimation result as sound, or may transmit the estimation result to an external device, e.g., a mobile device, a tablet PC, a personal computer, etc., through a communication module provided in the main body.

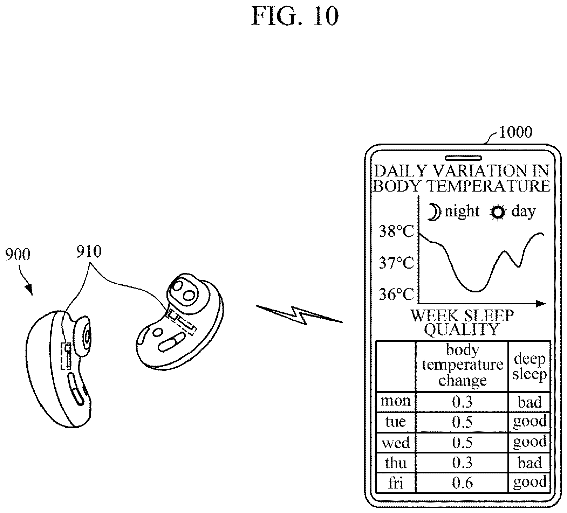

Referring to FIG. 10, the electronic device may be implemented by a combination of an ear-wearable device and a mobile device such as a smartphone. However, this is merely an example, and various combinations of electronic devices may be provided. For example, a processor for estimating body temperature may be mounted in a main body of a mobile device 1000. Upon receiving a request for measuring body temperature, the processor of the mobile device 1000 may control a communication interface to communicate with a communication module mounted in the main body of the wearable device 900, to obtain data, e.g., heat flux and surface temperature of an object, by using the sensor 910. Further, upon receiving data, such as the heat flux, the surface temperature of the object, etc., from the wearable device 900, the processor may estimate body temperature and may output an estimation result and body temperature information to the display of the mobile device 1000 through an output interface as illustrated herein. For example, in response to a user's request, the processor may display and provide not only a current estimated body temperature value, but also continuous estimated body temperature values over time on the display for the user. In addition, the processor may display a body temperature variation, for example, a body temperature change during a day, in a graph on the display, and may also display information on sleep quality according to the body temperature change on the display.

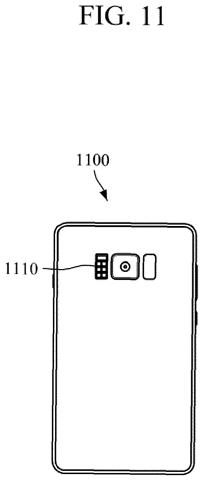

Referring to FIG. 11, the electronic device may be implemented as a mobile device 1100 such as a smartphone.

The mobile device 1100 may include a housing and a display panel. The housing may form an outer appearance of the mobile device 1100. The housing has a first surface, on which a display panel and a cover glass may be disposed sequentially, and the display panel may be exposed to the outside through the cover glass. A sensor 1110, a camera module and/or an infrared sensor, and the like may be disposed on a second surface of the housing.

For example, a plurality of temperature sensors for obtaining data from a user may be disposed on a rear surface of the mobile device 1100, and a fingerprint sensor disposed on the front surface of the mobile device 1100, a power button or a volume button disposed on a side surface thereof, sensors disposed on other positions of the front and rear surfaces of the mobile device 1100, and the like may be provided to estimate a user's body temperature.

In addition, when a user transmits a request for estimating body temperature by executing an application and the like installed in the mobile device 1100, the mobile device 1100 may obtain data by using the sensor 1110 (e.g., heat flux sensor), and may estimate the body temperature and may provide the estimated value to the user as image and/or sound by using the processor in the mobile device 1100.

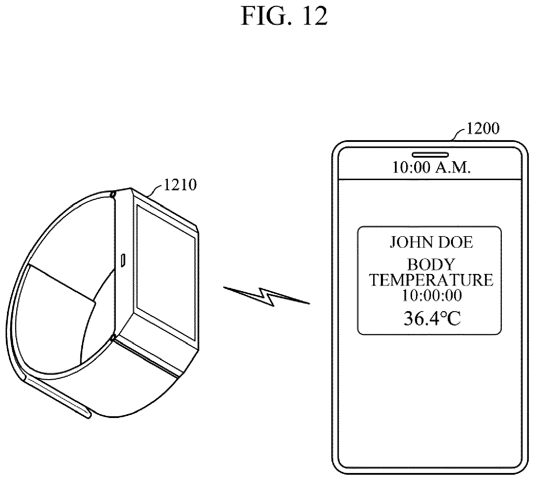

Referring to FIG. 12, the electronic device may be implemented as a combination of a wristwatch-type wearable device and a mobile device such as a smartphone. For example, a memory, a communication interface, and a processor for estimating body temperature may be mounted in a main body of a mobile device 1200. Upon receiving a request for measuring body temperature, the processor of the mobile device 1200 may control the communication interface to communicate with a communication module mounted in a main body of the wearable device 1210, to obtain data through the communication interface. Further, upon receiving data, such as heat flux, first temperature, and the like from the wearable device, the processor may estimate body temperature and output an estimation result to the display of the mobile device through an output interface as illustrated herein.

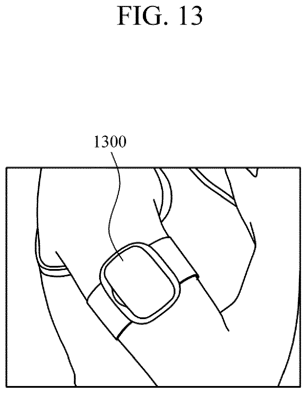

Referring to FIG. 13, an electronic device 1300 may be implemented as a patchtype device.

For example, the electronic device 1300 may be fixed to a body measurement location (e.g., upper arm, chest, etc.) by a strap, to measure a user's body temperature. In this case, the electronic device 1300 may provide the user with an estimated body temperature as sound or through a display, or may transmit the estimated body temperature to an external device, e.g., a mobile device, a tablet PC, other medical device, etc., through a communication module provided in the electronic device 1300.

While not restricted thereto, an example embodiment can be embodied as computer-readable code on a computer-readable recording medium. The computer-readable recording medium is any data storage device that can store data that can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an example embodiment may be written as a computer program transmitted over a computer-readable transmission medium, such as a carrier wave, and received and implemented in general-use or special-purpose digital computers that execute the programs. Moreover, it is understood that in example embodiments, one or more units of the above-described apparatuses and devices can include circuitry, a processor, a microprocessor, etc., and may execute a computer program stored in a computer-readable medium.

**Claims**

1. A sensor device (100, 600, 800, 900, 1210, 1300) comprising:

a first temperature sensor (111) configured to measure a first voltage (V1) when the sensor device (100, 600, 800, 900, 1210, 1300) is in contact with a user;
a second temperature sensor (112) disposed apart from the first temperature sensor (111) in a thickness direction of the sensor device (100), and configured to measure a second voltage (V2) when the sensor device (100) is in contact with the user; **characterized by**:

an amplifier (113) configured to amplify a voltage difference ($\Delta V$) between the first voltage (V1) and the second voltage (V2), and output the amplified voltage difference ($A \times \Delta V$) as a value that represents a body temperature ($T_{core}$) of the user.

2. The sensor device (100, 600, 800, 900, 1210, 1300) of claim 1, wherein the first temperature sensor (111), the second temperature sensor (112), and the amplifier (113) are included in analog front-end of the sensor device (100, 600, 800, 900, 1210, 1300), and the sensor device (100) further comprises:

an analog-to-digital, A/D, converter configured to convert the amplified voltage difference ($A \times \Delta V$) in an analog format to a digital signal; and
at least one processor (114, 120, 620) configured to determine the body temperature ($T_{core}$) of the user based on the digital signal.

3. The sensor device (100, 600, 800, 900, 1210, 1300) of claim 1 being wearable, and further comprising:

an analog-to-digital, A/D, converter configured to convert the amplified voltage difference ($A \times \Delta V$) in an analog format to a digital signal; and
at least one processor (114, 120, 620) configured to estimate the body temperature ($T_{core}$) of the user based on the digital signal representing the amplified voltage difference ($A \times \Delta V$).

4. The sensor device (100, 600, 800, 900, 1210, 1300) of claim 3, wherein a length of a space between the first temperature sensor (111) and the second temperature sensor (112) in the thickness direction is in a range from 0.1 mm to 5 mm.

5. The sensor device (100, 600, 800, 900, 1210, 1300) of claim 3 or 4, further comprising:
a thermally insulating material that is disposed between the first temperature sensor (111) and the second temperature sensor (112) in the thickness direction of the wearable device (100, 600, 800, 900, 1210, 1300).

6. The sensor device (100, 600, 800, 900, 1210, 1300) of claim 5, wherein the thermally insulating material has a conductivity of 0.1 W/mK or less.

7. The sensor device (100, 600, 800, 900, 1210, 1300) of claim 6, wherein the thermally insulating material is air or a polyurethane foam.

8. The sensor device (100, 600, 800, 900, 1210, 1300) of one of claims 3 to 7, wherein the first temperature sensor (111) and the second temperature sensor (112) are connected to have a Wheatstone bridge configuration.

9. The sensor device (100, 600, 800, 900, 1210, 1300) of one of claims 3 to 8, wherein at least one of the first temperature sensor (111) and the second temperature sensor (112) is a thermistor.

10. The sensor device (100, 600, 800, 900, 1210, 1300) of one of claims 3 to 9, wherein the at least one processor (114, 120 620) is further configured to convert the amplified voltage difference ($A \times \Delta V$) into a temperature difference ($\Delta T$), and estimate the body temperature ($T_{core}$) based on the temperature difference ($\Delta T$) corresponding to the amplified voltage difference ($A \times \Delta V$).

11. The sensor device (100, 600, 800, 900, 1210, 1300) of one of claims 3 to 10, further comprising a display (641) configured to display the body temperature ($T_{core}$) of the user.

12. A method of measuring a body temperature ($T_{core}$) using a wearable device (100, 600, 800, 900, 1210, 1300), the method comprising:

measuring (710) a first voltage (V1) by a first temperature sensor (111) when the wearable device (100, 600, 800, 900, 1210, 1300) is in contact with a user;
measuring (720) a second voltage (V2) by a second temperature sensor (112) that is disposed apart from the first temperature sensor (111) in a thickness direction of the wearable device (100, 600, 800, 900, 1210, 1300), when the wearable device (100, 600, 800, 900, 1210, 1300) is in contact with the user;
**characterized by**:

amplifying (730) a voltage difference ($\Delta V$) between the first voltage (V1) and the second voltage (V2);
converting (740) the amplified voltage difference (Ax$\Delta V$) in an analog format to a digital signal and
estimating (760) the body temperature ($T_{core}$) of the user based on the digital signal representing the amplified voltage difference (Ax$\Delta V$).

**13.** The method of claim 12, further comprising:

converting the amplified voltage difference (Ax$\Delta V$) into a temperature difference ($\Delta T$); and
estimating the body temperature ($T_{core}$) based on the temperature difference ($\Delta T$) corresponding to the amplified voltage difference (Ax$\Delta V$).

**14.** The method of claim 12 or 13, further comprising:

generating a conversion model based on a first temperature (T1) corresponding to the first voltage (V1), the first voltage (V1), and an external supply voltage;
converting the amplified voltage difference (Ax$\Delta V$) into a temperature difference ($\Delta T$) via the conversion model; and
estimating the body temperature ($T_{core}$) based on the temperature difference ($\Delta T$) corresponding to the amplified voltage difference (Ax$\Delta V$).

**15.** The method of one of claims 12 to 14, further comprising:

generating a conversion model based on a second temperature (T2) corresponding to the second voltage (V2), the second voltage (V2), and an external supply voltage;
converting the amplified voltage difference (Ax$\Delta V$) into a temperature difference ($\Delta T$) via the conversion model; and
estimating the body temperature ($T_{core}$) based on the temperature difference ($\Delta T$) corresponding to the amplified voltage difference (Ax$\Delta V$).

**16.** The method of one of claims 12 to 15, further comprising:

identifying a thermal coefficient of resistivity ($\beta_{ins}$) of a thermally insulating material disposed between the first temperature sensor (111) and the second temperature sensor (112) in the thickness direction; and
estimating the body temperature ($T_{core}$) of the user based on the amplified voltage difference (Ax$\Delta V$) and the thermal coefficient of resistivity ($\beta_{ins}$) of the thermally insulating material.

**17.** The method of claim 16, wherein the thermally insulating material has a conductivity of 0.1 W/mK or less.

**18.** The method of claim 17, wherein the thermally insulating material is air or a polyurethane foam.

**Patentansprüche**

**1.** Eine Sensorvorrichtung (100, 600, 800, 900, 1210, 1300), umfassend:

einen ersten Temperatursensor (111), der dazu konfiguriert ist, eine erste Spannung (V1) zu messen, wenn die Sensorvorrichtung (100, 600, 800, 900, 1210, 1300) in Kontakt mit einem Benutzer ist; einen zweiten Temperatursensor (112), der in einer Dickenrichtung der Sensorvorrichtung (100) von dem ersten Temperatursensor (111) entfernt angeordnet ist und dazu konfiguriert ist, eine zweite Spannung (V2) zu messen, wenn die Sensorvorrichtung (100) in Kontakt mit dem Benutzer ist;
**gekennzeichnet durch**:

einen Verstärker (113), der dazu konfiguriert ist, eine Spannungsdifferenz ($\Delta$V) zwischen der ersten Spannung (V1) und der zweiten Spannung (V2) zu verstärken und die verstärkte Spannungsdifferenz (Ax$\Delta$V) als einen Wert auszugeben, der eine Körpertemperatur ($T_{core}$) des Benutzers darstellt.

2. Sensorvorrichtung (100, 600, 800, 900, 1210, 1300) nach Anspruch 1, wobei der erste Temperatursensor (111), der zweite Temperatursensor (112) und der Verstärker (113) in einem analogen Front-End der Sensorvorrichtung (100, 600, 800, 900, 1210, 1300) enthalten sind, und die Sensorvorrichtung (100) des Weiteren umfasst:

einen Analog-Digital-Wandler (A/D), der dazu konfiguriert ist, die verstärkte Spannungsdifferenz (Ax$\Delta$V) in einem analogen Format in ein digitales Signal umzuwandeln; und
mindestens einen Prozessor (114, 120, 620), der dazu konfiguriert ist, die Körpertemperatur ($T_{core}$) des Benutzers auf der Grundlage des digitalen Signals zu bestimmen.

3. Sensorvorrichtung (100, 600, 800, 900, 1210, 1300) nach Anspruch 1, die tragbar ist und des Weiteren umfasst:

einen Analog-Digital-Wandler (A/D), der dazu konfiguriert ist, die verstärkte Spannungsdifferenz (Ax$\Delta$V) in einem analogen Format in ein digitales Signal umzuwandeln; und
mindestens einen Prozessor (114, 120, 620), der dazu konfiguriert ist, die Körpertemperatur ($T_{core}$) des Benutzers auf der Grundlage des digitalen Signals zu schätzen, das die verstärkte Spannungsdifferenz (Ax$\Delta$V) darstellt.

4. Sensorvorrichtung (100, 600, 800, 900, 1210, 1300) nach Anspruch 3, wobei eine Länge eines Zwischenraums zwischen dem ersten Temperatursensor (111) und dem zweiten Temperatursensor (112) in der Dickenrichtung in einem Bereich von 0,1 mm bis 5 mm liegt.

5. Sensorvorrichtung (100, 600, 800, 900, 1210, 1300) nach Anspruch 3 oder 4, des Weiteren umfassend:
ein wärmeisolierendes Material, das zwischen dem ersten Temperatursensor (111) und dem zweiten Temperatursensor (112) in der Dickenrichtung der tragbaren Vorrichtung (100, 600, 800, 900, 1210, 1300) angeordnet ist.

6. Sensorvorrichtung (100, 600, 800, 900, 1210, 1300) nach Anspruch 5, wobei das wärmeisolierende Material eine Leitfähigkeit von 0,1 W/mK oder weniger aufweist.

7. Sensorvorrichtung (100, 600, 800, 900, 1210, 1300) nach Anspruch 6, wobei das wärmeisolierende Material Luft oder ein Polyurethanschaum ist.

8. Sensorvorrichtung (100, 600, 800, 900, 1210, 1300) nach einem der Ansprüche 3 bis 7, wobei der erste Temperatursensor (111) und der zweite Temperatursensor (112) so angeschlossen sind, dass sie eine Wheatstone-Brücken-Konfiguration aufweisen.

9. Sensorvorrichtung (100, 600, 800, 900, 1210, 1300) nach einem der Ansprüche 3 bis 8, wobei mindestens einer von dem ersten Temperatursensor (111) und dem zweiten Temperatursensor (112) ein Thermistor ist.

10. Sensorvorrichtung (100, 600, 800, 900, 1210, 1300) nach einem der Ansprüche 3 bis 9, wobei der mindestens eine Prozessor (114, 120, 620) des Weiteren dazu konfiguriert ist, die verstärkte Spannungsdifferenz (Ax$\Delta$V) in eine Temperaturdifferenz ($\Delta$T) umzuwandeln und die Körpertemperatur ($T_{core}$) auf der Grundlage der Temperaturdifferenz ($\Delta$T) entsprechend der verstärkten Spannungsdifferenz (Ax$\Delta$V) zu schätzen.

11. Sensorvorrichtung (100, 600, 800, 900, 1210, 1300) nach einem der Ansprüche 3 bis 10, des Weiteren umfassend eine Anzeige (641), die dazu konfiguriert ist, die Körpertemperatur ($T_{core}$) des Benutzers anzuzeigen.

12. Verfahren zum Messen einer Körpertemperatur ($T_{core}$) unter Verwendung einer tragbaren Vorrichtung (100, 600, 800, 900, 1210, 1300), wobei das Verfahren umfasst:

Messen (710) einer ersten Spannung (V1) durch einen ersten Temperatursensor (111), wenn die tragbare Vorrichtung (100, 600, 800, 900, 1210, 1300) in Kontakt mit einem Benutzer ist;
Messen (720) einer zweiten Spannung (V2) durch einen zweiten Temperatursensor (112), der von dem ersten Temperatursensor (111) in einer Dickenrichtung der tragbaren Vorrichtung (100, 600, 800, 900, 1210, 1300) entfernt angeordnet ist, wenn die tragbare Vorrichtung (100, 600, 800, 900, 1210, 1300) in Kontakt mit dem

13

Benutzer ist;
**gekennzeichnet durch:**

Verstärken (730) einer Spannungsdifferenz ($\Delta$V) zwischen der ersten Spannung (V1) und der zweiten Spannung (V2);
Umwandeln (740) der verstärkten Spannungsdifferenz (Ax$\Delta$V) in einem analogen Format in ein digitales Signal und Schätzen (760) der Körpertemperatur ($T_{core}$) des Benutzers auf der Grundlage des digitalen Signals, das die verstärkte Spannungsdifferenz (Ax$\Delta$V) darstellt.

**13.** Verfahren nach Anspruch 12, des Weiteren umfassend:

Umwandeln der verstärkten Spannungsdifferenz (Ax$\Delta$V) in eine Temperaturdifferenz ($\Delta$T); und
Schätzen der Körpertemperatur ($T_{core}$) auf der Grundlage der Temperaturdifferenz ($\Delta$T) entsprechend der verstärkten Spannungsdifferenz (Ax$\Delta$V).

**14.** Verfahren nach Anspruch 12 oder 13, des Weiteren umfassend:

Erzeugen eines Umwandlungsmodells auf der Grundlage einer ersten Temperatur (T1) entsprechend der ersten Spannung (V1), der ersten Spannung (V1), und einer externen Versorgungsspannung;
Umwandeln der verstärkten Spannungsdifferenz (Ax$\Delta$V) in eine Temperaturdifferenz ($\Delta$T) mittels des Umwandlungsmodells; und
Schätzen der Körpertemperatur ($T_{core}$) auf der Grundlage der Temperaturdifferenz ($\Delta$T) entsprechend der verstärkten Spannungsdifferenz (Ax$\Delta$V).

**15.** Verfahren nach einem der Ansprüche 12 bis 14, des Weiteren umfassend:

Erzeugen eines Umwandlungsmodells auf der Grundlage einer zweiten Temperatur (T2) entsprechend der zweiten Spannung (V2), der zweiten Spannung (V2), und einer externen Versorgungsspannung;
Umwandeln der verstärkten Spannungsdifferenz (Ax$\Delta$V) in eine Temperaturdifferenz ($\Delta$T) mittels des Umwandlungsmodells; und
Schätzen der Körpertemperatur ($T_{core}$) auf der Grundlage der Temperaturdifferenz ($\Delta$T) entsprechend der verstärkten Spannungsdifferenz (Ax$\Delta$V).

**16.** Verfahren nach einem der Ansprüche 12 bis 15, des Weiteren umfassend:

Identifizieren eines Wärmewiderstandskoeffizienten ($\beta_{ins}$) eines wärmeisolierenden Materials, das zwischen dem ersten Temperatursensor (111) und dem zweiten Temperatursensor (112) in der Dickenrichtung angeordnet ist; und
Schätzen der Körpertemperatur ($T_{core}$) des Benutzers auf der Grundlage der verstärkten Spannungsdifferenz (Ax$\Delta$V) und des Wärmewiderstandskoeffizienten ($\beta_{ins}$) des wärmeisolierenden Materials.

**17.** Verfahren nach Anspruch 16, wobei das wärmeisolierende Material eine Leitfähigkeit von 0,1 W/mK oder weniger aufweist.

**18.** Verfahren nach Anspruch 17, wobei das wärmeisolierende Material Luft oder ein Polyurethanschaum ist.

**Revendications**

**1.** Dispositif de capteurs (100, 600, 800, 900, 1210, 1300) comprenant :

un premier capteur de température (111) configuré pour mesurer une première tension (V1) lorsque le dispositif de capteurs (100, 600, 800, 900, 1210, 1300) est en contact avec un utilisateur ;
un second capteur de température (112) disposé à l'écart du premier capteur de température (111) dans une direction d'épaisseur du dispositif de capteurs (100), et configuré pour mesurer une seconde tension (V2) lorsque le dispositif de capteurs (100) est en contact avec l'utilisateur ;
**caractérisé par** :
un amplificateur (113) configuré pour amplifier une différence de tension ($\Delta$V) entre la première tension (V1) et la seconde tension (V2), et délivrer en sortie la différence de tension amplifiée (AxΔV) en tant que valeur qui représente une température corporelle (T$_{core}$) de l'utilisateur.

2. Dispositif de capteurs (100, 600, 800, 900, 1210, 1300) selon la revendication 1, dans lequel le premier capteur de température (111), le second capteur de température (112), et l'amplificateur (113) sont compris dans une extrémité avant analogique du dispositif de capteurs (100, 600, 800, 900, 1210, 1300), et le dispositif de capteurs (100) comprend en outre :

un convertisseur analogique-numérique, A/N, configuré pour convertir la différence de tension amplifiée (AxΔV) dans un format analogique en un signal numérique ; et
au moins un processeur (114, 120, 620) configuré pour déterminer la température corporelle (T$_{core}$) de l'utilisateur sur la base du signal numérique.

3. Dispositif de capteurs (100, 600, 800, 900, 1210, 1300) selon la revendication 1, qui peut être porté sur soi, et comprenant en outre :

un convertisseur analogique-numérique, A/N, configuré pour convertir la différence de tension amplifiée (AxΔV) dans un format analogique en un signal numérique ; et
au moins un processeur (114, 120, 620) configuré pour estimer la température corporelle (T$_{core}$) de l'utilisateur sur la base du signal numérique représentant la différence de tension amplifiée (AxΔV).

4. Dispositif de capteurs (100, 600, 800, 900, 1210, 1300) selon la revendication 3, dans lequel une longueur d'un espace entre le premier capteur de température (111) et le second capteur de température (112) dans la direction d'épaisseur est comprise entre 0,1 mm et 5 mm.

5. Dispositif de capteurs (100, 600, 800, 900, 1210, 1300) selon la revendication 3 ou 4, comprenant en outre :
un matériau thermiquement isolant qui est disposé entre le premier capteur de température (111) et le second capteur de température (112) dans la direction d'épaisseur du dispositif à porter sur soi (100, 600, 800, 900, 1210, 1300).

6. Dispositif de capteurs (100, 600, 800, 900, 1210, 1300) selon la revendication 5, dans lequel le matériau thermiquement isolant a une conductivité de 0,1 W/mK ou moins.

7. Dispositif de capteurs (100, 600, 800, 900, 1210, 1300) selon la revendication 6, dans lequel le matériau thermiquement isolant est de l'air ou une mousse de polyuréthane.

8. Dispositif de capteurs (100, 600, 800, 900, 1210, 1300) selon l'une des revendications 3 à 7, dans lequel le premier capteur de température (111) et le second capteur de température (112) sont connectés pour avoir une configuration de pont de Wheatstone.

9. Dispositif de capteurs (100, 600, 800, 900, 1210, 1300) selon l'une des revendications 3 à 8, dans lequel au moins l'un du premier capteur de température (111) et du second capteur de température (112) est une thermistance.

10. Dispositif de capteurs (100, 600, 800, 900, 1210, 1300) selon l'une des revendications 3 à 9, dans lequel le au moins un processeur (114, 120, 620) est en outre configuré pour convertir la différence de tension amplifiée (AxΔV) en une différence de température (ΔT), et estimer la température corporelle (T$_{core}$) sur la base de la différence de température (ΔT) correspondant à la différence de tension amplifiée (AxΔV).

11. Dispositif de capteurs (100, 600, 800, 900, 1210, 1300) selon l'une des revendications 3 à 10, comprenant en outre un affichage (641) configuré pour afficher la température corporelle (T$_{core}$) de l'utilisateur.

12. Procédé de mesure d'une température corporelle (T$_{core}$) à l'aide d'un dispositif à porter sur soi (100, 600, 800, 900, 1210, 1300), le procédé comprenant :

la mesure (710) d'une première tension (V1) par un premier capteur de température (111) lorsque le dispositif à porter sur soi (100, 600, 800, 900, 1210, 1300) est en contact avec un utilisateur ;
la mesure (720) d'une seconde tension (V2) par un second capteur de température (112) qui est disposé à l'écart du premier capteur de température (111) dans une direction d'épaisseur du dispositif à porter sur soi (100, 600, 800, 900, 1210, 1300), lorsque le dispositif à porter sur soi (100, 600, 800, 900, 1210, 1300) est en contact avec l'utilisateur ;
**caractérisé par** :

l'amplification (730) d'une différence de tension (ΔV) entre la première tension (V1) et la seconde tension (V2) ;
la conversion (740) de la différence de tension amplifiée (AxΔV) dans un format analogique en un signal numérique ;
et
l'estimation (760) la température corporelle (T$_{core}$) de l'utilisateur sur la base du signal numérique représentant la différence de tension amplifiée (AxΔV).

13. Procédé selon la revendication 12, comprenant en outre :

la conversion de la différence de tension amplifiée (AxΔV) en une différence de température (ΔT) ; et
l'estimation de la température corporelle (T$_{core}$) sur la base de la différence de température (ΔT) correspondant à la différence de tension amplifiée (AxΔV).

14. Procédé selon la revendication 12 ou 13, comprenant en outre :

la génération d'un modèle de conversion sur la base d'une première température (T1) correspondant à la première tension (V1), de la première tension (V1), et d'une tension d'alimentation externe ;
la conversion de la différence de tension amplifiée (AxΔV) en une différence de température (ΔT) via le modèle de conversion ; et
l'estimation de la température corporelle (T$_{core}$) sur la base de la différence de température (ΔT) correspondant à la différence de tension amplifiée (AxΔV).

15. Procédé selon l'une des revendications 12 à 14, comprenant en outre :

la génération d'un modèle de conversion sur la base d'une seconde température (T2) correspondant à la seconde tension (V2), de la seconde tension (V2), et d'une tension d'alimentation externe ;
la conversion de la différence de tension amplifiée (AxΔV) en une différence de température (ΔT) via le modèle de conversion ; et
l'estimation de la température corporelle (T$_{core}$) sur la base de la différence de température (ΔT) correspondant à la différence de tension amplifiée (AxΔV).

16. Procédé selon l'une des revendications 12 à 15, comprenant en outre :

l'identification d'un coefficient de résistivité thermique (β$_{ins}$) d'un matériau thermiquement isolant disposé entre le premier capteur de température (111) et le second capteur de température (112) dans la direction d'épaisseur ;
et
l'estimation de la température corporelle (T$_{core}$) de l'utilisateur sur la base de la différence de tension amplifiée (AxΔV) et du coefficient de résistivité thermique (β$_{ins}$) du matériau thermiquement isolant.

17. Procédé selon la revendication 16, dans lequel le matériau thermiquement isolant a une conductivité de 0,1 W/mK ou moins.

18. Procédé selon la revendication 17, dans lequel le matériau thermiquement isolant est de l'air ou une mousse de polyuréthane.

FIG. 1

```
                              ┌─100
┌ ─ ─ ─ ─ ─ ─ ─ ─ ─ ─ ─ ─ ─ ─ ┐
│               ┌─110          │
│  ┌─────────────────────────┐ │
│  │    HEAT FLUX SENSOR     │ │
│  └─────────────────────────┘ │
│              ↕               │
│               ┌─120          │
│  ┌─────────────────────────┐ │
│  │       PROCESSOR         │ │
│  └─────────────────────────┘ │
└ ─ ─ ─ ─ ─ ─ ─ ─ ─ ─ ─ ─ ─ ─ ┘
```

# FIG. 2

FIG. 3

```
                              ┌─110
        ┌──────────────────────────┐
        │  ┌──────────────────────┐ │
        │  │       SECOND         │ │
        │  │   TEMPERATURE        │─┼─ 112
        │  │       SENSOR         │ │
        │  ├──────────────────────┤ │
        │  │      THERMALLY       │ │
        │  │     CONDUCTIVE       │─┼─ 310
        │  │      MATERIAL        │ │
        │  ├──────────────────────┤ │
        │  │        FIRST         │ │
        │  │    TEMPERATURE       │─┼─ 111
        │  │       SENSOR         │ │
        │  └──────────────────────┘ │
        └──────────────────────────┘
```

# FIG. 4

# FIG. 5

# FIG. 6

# FIG. 7

```
                        ┌─────────┐
                        │  START  │
                        └─────────┘
                             │
           ┌─────────────────┴─────────────────┐
           │ ⌐710                              │ ⌐720
           ▼                                   ▼
  ┌──────────────────┐              ┌──────────────────┐
  │  MEASURE FIRST   │              │  MEASURE SECOND  │
  │   TEMPERATURE    │              │   TEMPERATURE    │
  └──────────────────┘              └──────────────────┘
           │                                   │
           └─────────────────┬─────────────────┘
                             │ ⌐730
                             ▼
              ┌───────────────────────────┐
              │   AMPLIFY VOLTAGE         │
              │   DIFFERENCE BETWEEN      │
              │   FIRST VOLTAGE AND       │
              │   SECOND VOLTAGE          │
              └───────────────────────────┘
                             │ ⌐740
                             ▼
              ┌───────────────────────────┐
              │   CONVERT AMPLIFIED       │
              │ VOLTAGE DIFFERENCE INTO   │
              │ TEMPERATURE DIFFERENCE    │
              └───────────────────────────┘
                             │ ⌐750
                             ▼
              ┌───────────────────────────┐
              │    CALCULATE AND          │
              │   OUTPUT HEAT FLUX        │
              └───────────────────────────┘
                             │ ⌐760
                             ▼
              ┌───────────────────────────┐
              │    ESTIMATE BODY          │
              │    TEMPERATURE            │
              └───────────────────────────┘
                             │
                             ▼
                        ┌─────────┐
                        │   END   │
                        └─────────┘
```

# FIG. 8

# FIG. 9

# FIG. 10

DAILY VARIATION IN BODY TEMPERATURE

☾ night  ☼ day

38°C
37°C
36°C

WEEK SLEEP QUALITY

|  | body temperature change | deep sleep |
|---|---|---|
| mon | 0.3 | bad |
| tue | 0.5 | good |
| wed | 0.5 | good |
| thu | 0.3 | bad |
| fri | 0.6 | good |

FIG. 11

## FIG. 12

FIG. 13

1300

**EP 4 300 062 B1**

## REFERENCES CITED IN THE DESCRIPTION

### Patent documents cited in the description

- JP 561120026 A
- US 2022042856 A1